# EUROPEAN PATENT SPECIFICATION

(11) **EP 2 143 394 B1**
(45) Date of publication and mention of the grant of the patent: **27.05.2015**
(21) Application number: 08761543.1
(22) Date of filing: 30.04.2008
(51) Int. Cl.: A61B 18/14, A61B 18/00

(54) **APPLICATOR DEVICE FOR ABLATION BY RADIOFREQUENCY OF BIOLOGICAL TISSUES**
APPLIKATORVORRICHTUNG ZUR HOCHFREQUENZABLATION BIOLOGISCHER GEWEBE
DISPOSITIF APPLICATEUR POUR L'ABLATION PAR RADIOFRÉQUENCE DE TISSUS BIOLOGIQUES

(30) Priority: 30.04.2007 ES 200701223
(43) Date of publication of application: 13.01.2010
(73) Proprietor: Universidad Politecnica de Valencia, 46022 Valencia (ES); Universidad de Zaragoza, 50005 Zaragoza (ES)
(72) Inventor: BERJANO ZANÓN, Enrique, 46022 Valencia (ES); BURDIO PINILLA, Fernando, 46022 Valencia (ES); GÜEMES SÁNCHEZ, Antonio, 46022 Valencia (ES); NAVARRO GONZALO, Ana, 46022 Valencia (ES)
(74) Representative: Pons Ariño, Angel
(86) International application number: PCT/ES2008/000297
(87) International publication number: WO 2008/132259

(56) References cited:
- EP-A1- 1 149 564
- WO-A1-00/09208
- WO-A1-03/026525
- WO-A2-97/06739
- US-A1- 2006 122 593
- US-A1- 2006 178 666
- NI Y. ET AL.: 'A novel "cooled-wet" electrode for RF ablation' EUROPEAN RADIOLOGY vol. 10, 2000, pages 852 - 854, XP008117299
- YI MIAO ET AL.: 'A Comparative Study on Validation of a Novel Cooled-Wet Electrode for Radiofrequency Lisee Ablation' INVESTIGATE RADIOLOGY vol. 35, no. 7, 2000, pages 438 - 443

## Description

### OBJECT OF THE INVENTION

The present invention relates in general to the therapeutic treatment of biological tissues, and to the use of thermal energy for the ablation of tumours.

### BACKGROUND OF THE INVENTION

The ablation of tumours by thermal damage to the unwanted cells by the application of radiofrequency currents is not new. When the frequency of the electrical current exceeds 10 kHz no pain or muscular contraction occurs, but instead ionic friction is generated which turns into heat. Radiofrequency ablation uses frequency currents between 100 kHz and 2 MHz. The increase in temperature in tissues above 50ºC leads to the rupture of proteins and membranes, which results in necrosis by coagulation and subsequent cell death. The use of radiofrequency ablation to destroy tumours is based on the doctor's skill in inserting one or several electrodes (also called applicators) in the tumour by guided computed tomography or ultrasounds. There has recently been great interest in radiofrequency ablation guided by image techniques as a new technique of minimally invasive therapy, especially for primary liver tumours or foci of metastasis, due to the high mortality and morbidity of standard surgical resection and the high number of patients who do not tolerate such an aggressive surgery.

In the most commonly used monopolar configuration, the current flows from an electrode with small dimensions called active electrode through the tissues towards a dispersive electrode (also called patch or plaque, of greater dimension, and located in the patient's back or thigh). During the conventional application of radiofrequency, the electrically power is fundamentally deposited in the tissue around the active electrode in a margin of a few millimetres, which produces heating exclusively localized in a narrow area around said electrode. This heat generated immediately adjacent to the electrode spreads through the rest of the tissue by thermal conduction. As a consequence, the application of radiofrequency involves a rapid increase in temperature (sometimes above 100ºC) in the tissue-active electrode interface, which causes the excessive desiccation of the tissue and formation of a necrosis by coagulation. The excessively desiccated cell tissue adheres to the electrode and forms an insulating cover which produces a fast and significant increase in electrical impedance. In these circumstances, the radiofrequency generator cannot distribute more power (this phenomenon is called "roll-off"). When this happens, it is not possible to deposit more energy and the total volume of the coagulated area is very limited, which may produce a lesion which does not include the entire tumour volume.

Different types of electrodes have been developed in order to avoid this problem. A strategy to minimize the problem of "roll-off' has been that adopted by cooled electrodes, such as that disclosed in the United States patent US-7077842-B1. This electrode is composed of two inner tubes connected in the distal part: one of them transports a cold saline fluid towards the distal point whilst the other returns that same fluid heated by the effect of heating of the tissue (which occurs in the same distal zone).

Also, for example, Spanish patent application ES-2229345 discloses a cooled electrode which includes a cavity in the main antenna or electrode which transports an infusion medium; it also has secondary antennas to house temperature sensors or administer radiofrequency energy.

There are other patents which disclose the use of conventional electrodes, cooled electrodes, and even groupings thereof, i.e. clusters such as those disclosed in United States patents US-6530922-B2 or US-6641580-B1. Patent US-6530922-B2 discloses a grouping or cluster of cooled electrodes; it also has a type of hook which protrudes laterally and whose function is to achieve a good anchoring with the tissue.

The use of this type of electrodes (*cooled electrodes*) achieves that the highly desiccated, even carbonized, area of tissue is not adjacent to the electrode, but instead moves to approximately 3 mm beyond the outer surface of the electrode, leaving a type of carbonized tissue of approximately 1 mm thickness. This localization of the carbonized tissue adapts well to the thermal profile of the tissue during the ablation with these electrodes, since the maximum temperature reached has been observed approximately 2.5 mm from the surface of the electrode. Beyond this point, the lesion is mainly created passively by heat conduction until equilibrium is reached. As a consequence, the cooled electrodes allow creating a greater coagulation volume with respect to conventional electrodes, but limited when it concerns ablating tumours of large dimensions.

Another strategy for increasing the coagulation volume is to infuse a saline solution within the tissue through the same active electrodes (they receive the name of wet electrodes). This strategy has been proposed in combination with monopolar or bipolar ablation electrodes, or in combination with expandable type electrodes (such as those disclosed in patent documents US-6660002-B1 and WO-2005/037118-A1). The result has been an increase in coagulation volume. Two mechanisms causing this event have been proposed: a) the high electrical conductivity of the saline solution -NaCl typically - produces an increase in the electrical conductivity of the infused tissue, which allows the deposition of a greater quantity of electrical power, and b) the infusion of fluid during the ablation improves heat conduction within the tissue, which permits the convection of the heat more effectively and rapidly on a large volume of tissue, thus avoiding the desiccation of tissue around the electrode. With these perfused electrodes, greater coagulation volumes have been achieved than with cooled electrodes. However, they still have some limitations, in particular, in a monopolar configuration:
a) The boiling of the saline solution in the active electrode-tissue interface still occurs frequently when the power is programmed above 50 watts. As a consequence of the vaporization of the saline around the electrode, cavitation may occur in the tissue adjacent to the electrode. This effect may bring three consequences. On the one hand, it may prevent the subsequent infusion of saline in the tissue, which makes the efficacy of the ablation decrease with this type of electrodes. On the other, it may cause the reflux of saline through the cavity created by the electrode in its entry trajectory in the tissue. This may also cause the leaking of the hot solution towards the peritoneal cavity. Finally, it may lead to the creation of an irregular coagulation volume which does not adapt to the form of the tumour.
b) Although boiling of the saline is avoided, it is difficult to ensure a controlled infusion thereof beyond 4 or 5 mm from the surface of the cooled electrode. This is especially true when the density of the tissue to be infused is high, and if the saline is infused through lateral orifices, and not through an orifice centrally located in the point of the electrode. As a consequence, the carbonization of the tissue may appear at 4-5 mm distance to the electrode, and therefore, interrupt the power deposition. Electrodes have also been proposed simultaneously combining cooling and infusion of saline such as those disclosed in the United States patent US-6514251-B1 and in the patent application US-2006/0122593A1. These electrodes are called cooled-wet. The most outstanding element of all of them is that the saline serum is infused through orifices located in the same surface of the cooled electrode, i.e. at zero distance from said electrode.

Another type of applicator has been disclosed in document WO-A-97/06739 (also published WO 97/06857). In addition to being cooled, this electrode permits the infusion of saline solution. This saline solution may run both through the cavity of the main electrode and through the cavity of the secondary probes or antennas. The secondary antennas do not infuse saline serum towards the tissue, instead the serum flows internally through two lumens. In this way, the secondary antennas may act as cooled electrodes (obviously distributing energy through them), in addition to being able to house temperature sensors.

Patent application EP-1656900-A2 discloses an applicator in the form of a loop designed to resect polyps which has a needle which infuses a serum at a distance (it can be used to dampen the polyp before resection). However, the loop electrode of this patient is a dry electrode, i.e. it is not a cooled electrode. Furthermore, this needle is not electrically insulated, instead it is metal which also allows it to act as active electrode. Finally, patent application US2006/178666 describes a device for ablating tissue in the living body which includes an elongate member defining a longitudinal passage having a distal opening and a proximal opening dimensioned to pass a guide element where the elongate member includes an electrode disposed at a distal portion of the elongate member and configured to be energized with high frequency energy to ablate tissue; nonetheless this document is silent about the configuration of the device just describing the parts of said device and the method of carrying out an ablation of biological tissue by means of said device, moreover the device is characterized by having an helical member extending from the distal end of an internal lumen.

As a consequence, it is desirable to have a device for the therapeutic treatment of biological tissue which exceeds the limitations of the aforementioned devices, thus achieving lesions with a high coagulation volume.

### DESCRIPTION OF THE INVENTION

The invention relates to an applicator device for radiofrequency ablation of biological tissues in accordance with claim 1.

Preferred embodiments of the applicator device are defined in dependent claims.

The present invention provides an applicator device for radiofrequency ablation of biological tissues, particularly but not exclusively for hepatic tumours, which resolves the problems posed by the inventive subject included in the attached independent claim.

The present invention achieves this objective as it combines the effect of the cooling in the electrode -by a closed circuit of cooling fluid-, with the infusion of a saline solution in the tissue at a critical point between 1 and 5 mm distance to the surface of the electrode through one or several infusing needles. In this way, the device in the present invention makes it possible to create lesions with greater coagulation volumes than those achieved by the use of conventional electrodes, cooled electrodes, and even groupings thereof. Although electrodes have also been proposed which combine cooling and saline infusion, they are electrodes which infuse the saline solution from the surface of the cooled electrode, and not at a critical distance between 1 and 5 mm, which does not allow improving the aforementioned limitations.

The applicator device object of the invention minimizes the boiling of the saline fluid and the carbonization of the adjacent tissue, improving the electrical conductivity in the critical area between 1 and 5 mm; in this way lesions are achieved with a high coagulation volume.

In accordance with a first aspect of the invention, it relates to an applicator device for radiofrequency ablation of biological tissues which comprises:
- an electrode whose outer surface is covered with an insulating cover in a proximal portion, and has a conductive distal portion, whose electrode includes cooling means, and
- an infusion system with a distal end for infusing a conductive fluid (such as, for example, a saline solution) in said tissue, characterized in that said distal end of the infusion system has an electrically insulating outer cover and is located, in the infusion position of said fluid, at a distance between 1 and 5 mm from said outer surface of the conductive distal portion of the cooled electrode.

In accordance with a preferred embodiment of the applicator device of the invention, the infusion system comprises at least one needle -with electrically insulating cover- which, in the use position, is located fixed to the device, and a distal end of said needle is located at said distance between 1 and 5 mm from the outer surface of the distal portion of the electrode. In this embodiment the axial axis of said needle and that of the electrode are preferably parallel.

In accordance with another possible embodiment of the applicator device of the invention, the infusion system comprises at least one expandable tube with a distal end -whose outer surface has an electrically insulating cover-, and in that said distal end in the infusion position of the conductive fluid is at a distance between 1 and 5 mm from the outer surface of the distal portion of the electrode. In this case, said expandable tube preferably is located in a channel in the interior of the electrode.

The distal end of the infusion system is located, in the infusion position for the conductive fluid, at a longitudinal half of said conductive distal portion of the cooled electrode.

Although not forming part of the invention, a method of radiofrequency ablation of biological tissues is disclosed, which comprises:
- inserting in said biological tissue a conductive distal portion of an electrode,
- supplying high frequency electrical energy in said distal portion of the electrode,
- cooling said electrode continuously during said supply of energy and also optionally before said supply, and
- injecting in said biological tissue a conductive fluid at a distance between 1 and 5 mm from an outer surface of a conductive distal portion of said cooled electrode.

Preferably said fluid is inserted in the biological tissue by an infusion system, which is also inserted in the biological tissue and is externally covered by an electrically insulating cover, and simultaneously or previously the cooled electrode is inserted.

A single applicator can also be inserted in the biological tissue which comprises the electrode and an infusion system of said conductive fluid. In said case, said infusion system comprises at least one expandable tube - whose outer surface has an electrically insulating cover- and in that injecting the conductive fluid in the biological tissue comprises expanding said tube until a distal end of said tube is at a distance between 1 and 5 mm from the outer surface of the electrode.

The distal end of the infusion system is located at a longitudinal half of said conductive distal portion of the cooled electrode.

### BRIEF DESCRIPTION OF THE DRAWINGS

To complement the description being made, and in order to aid towards a better understanding of the characteristics of the invention, in accordance with preferred examples of practical embodiment thereof, a set of drawings is attached as an integral part of said description wherein the following has been represented with illustrative and non-limitative character:
Figure 1 shows a partial sectional view of an applicator device for ablation of biological tissues in accordance with a first possible embodiment thereof.
Figure 2 shows a partial sectional view of a second possible embodiment of the applicator device of the invention.
Figures 3 and 4 are two perspective views of the device of the invention according to the preferred embodiment shown in figure 2.
Figure 5 shows the effect of the saline infusion distance on the lesion volume (a), average distributed power (b), smaller or minimum diameters (c) and larger or maximum diameters (d).
Figure 6 represents the relative frequency of uncontrolled increases in impedance according to the ablation time and the infusion distance.

### DESCRIPTION OF A PREFERRED EMBODIMENT OF THE INVENTION

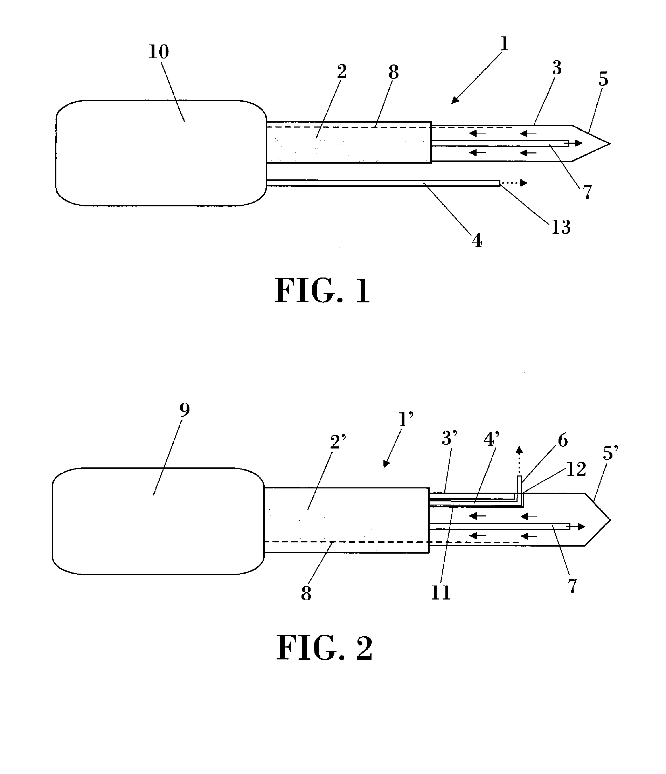

Figure 1 shows a cooled electrode with a saline infusion system assembled in parallel by a fixating system.

This applicator 1 contains a cooled electrode and a saline infusion system assembled in parallel by means of a fixation system 10 and at a distance between them which can vary between 1 and 5 mm.

The electrode's structure is as follows: it has an external diameter of approximately 1.8 mm. It is made of stainless steel and has a part covered with electrical insulator 2, and has a sharp point at its end 5. It can also have one or more thermocouples 8 located in the central part of the metal zone 3 and adhered to the interior of the electrode. The length of the metal zone should adapt to the length of the tissue area one wants to coagulate. The cooling system is based on a cannula 7 which is approximately 0.4 mm in diameter. In figure 1 the solid arrows indicate the direction of the cooled fluid which circulates internally through the cooled electrode. The broken arrow indicates the direction of the infused fluid directly in the tissue at a critical point between 1 and 5 mm from the cover of the cooled electrode.

Once a dispersive electrode (not represented) is located on the back or thigh of the patient and connected to a radiofrequency generator by a monopolar configuration, the operation is as follows: first, the cooled electrode is introduced in the desired position of the tissue. In second place, water or pre-cooled saline solution is infused (at a temperature, for example, of 0ºC) through the cannula 7 and at a flow of approximately 30 mL/minute. This is done, for example, by a peristaltic pump. The temperature of the metal zone 3 of the electrode is continuously monitored by the thermocouple 8 and displayed by the system of the radiofrequency generator (such as, for example, that marketed by Radionics Inc.) or by any independent system built for said purpose. When the temperature recorded by the thermocouple 8 reaches a value under 20ºC the distribution of radiofrequency energy starts through the metal part 3 following a specific energy deposition algorithm. During said energy distribution, the internal infusion of saline or pre-cooled water does not cease. The energy distribution algorithm may be controlled automatically or manually. There are different algorithm alternatives, but they are typically based on an energy distribution at maximum power (usually 200 watts) and an interruption -lasting between 10 and 60 seconds- in the case that the electrical impedance (measured between the metal part 3 and the dispersive electrode) exceeds a level -between 10 and 30 ohms- the initial impedance. In this configuration said interruption may also take place in the event that the temperature recorded in the thermocouple 8 should exceed 30ºC. Throughout the method, the cold liquid that flows through the cannula 7 is returned, now heated, towards a reservoir (not shown) for said purpose.

The objectives of the cooled electrode are:
- distributing the energy through its metal part directly towards the biological tissue;
- minimizing the carbonization of the tissue adjacent to the electrode; and,
- minimizing the boiling of the saline fluid diffused in the tissue adjacent to the electrode.

The saline infusion system is based on a cannula 4 of approximately 1 mm of diameter with insulating cover and assembled in parallel to the cooled electrode. The distance from the exit point of the saline from the cannula's 4 to the metal part 3 of the electrode may vary between 1 and 5 mm. The infusion system may be introduced in the tissue at the same time as the electrode or *a posteriori.* One minute before the power is distributed through the metal part 3 of the electrode, and also during said distribution, an infusion of isotonic (0.9%) or hypertonic (typically 20% NaCl) saline solution is performed through the cannula 4 at a flow of approximately 100 mL/hour.

The objectives of the saline infusion system are:
- improving the electrical conductivity of the tissue by its high NaCl concentration (it is a saline with a typical concentration between 0.9 and 40% NaCl); and,
- cooling the tissue located at a critical distance between 1 and 5 mm from the metal part 3 of the electrode in order to avoid the carbonization of said tissue.

For its part, the fixation system 10 maintains the cannula 4 of the infusion system at a specific distance -between 2 and 5 mm- from the outer part of the electrode. Different configurations are possible, the simplest would be based on a resin block that solidly joins the electrode and the cannula (4).

Figures 2-4 show a second embodiment of the invention, with a cooled electrode with expandable tubes of saline solution all forming a single applicator 1 '.

In this embodiment, a cooled electrode and infusion system are incorporated in the same element which serves as a handle 9.

The objectives in this case are the same as those stated for the embodiment shown in figure 1.

In this case, the maximum outer diameter (it could be less) is approximately 2.2 mm. The electrode is made from stainless steel and has a part with insulating cover 2' and a metal part 3' finished in a point 5'. The length of the metal zone 3' should adapt to the length of the tissue area one wants to coagulate. It has an internal infusion T cannula 7'-of approximately 0.4 mm in diameter. There is also a thermocouple 8' in the inner wall of the electrode at the average distance of the metal part 3'.

The operation in this case is the same as has been described for the device 1 shown in figure 1.

In this case, the saline infusion system comprises one or more expandable tubes of saline infusion 6. The objectives of this saline infusion system are the same as that described for the embodiment of figure 1, and also the following:
- avoiding a double puncture in the tissue, as required if the first embodiment is used; and,
- infusing the saline (isotonic or hypertonic) at a specific distance with greater accuracy.

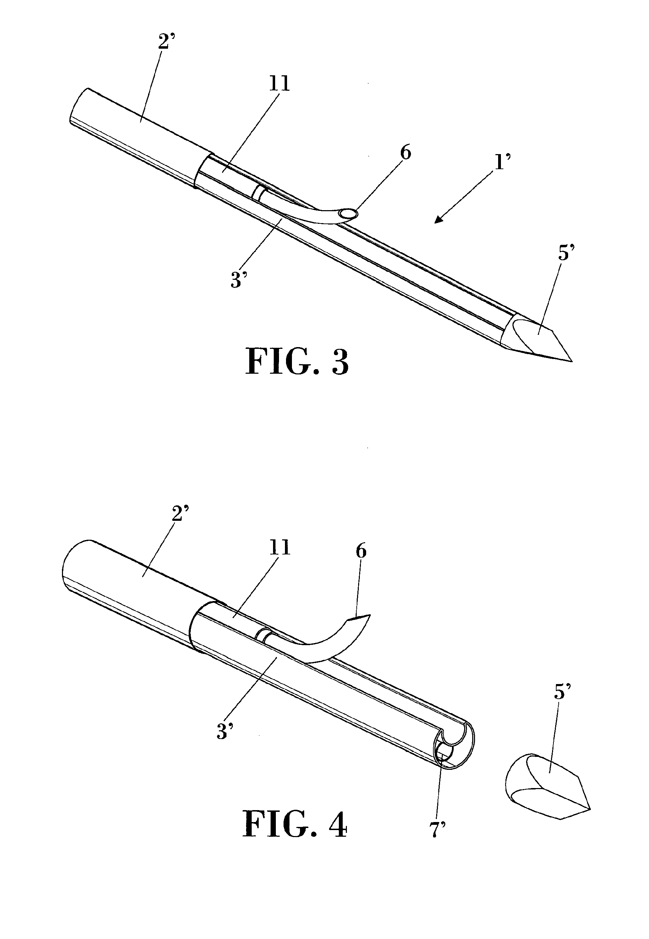

The system comprises a saline infusion cannula 4' which is incorporated within a rigid channel 11 located inside the electrode. It has an approximate diameter of 1 mm. This cannula 4' ends up exiting the electrode through a lateral orifice 12 located in the centre of the metal part 3'. Through this orifice the cannula exits the electrode forming an external cannula 6 that can adopt different forms such as straight (shown in figure 2), or J-shaped (shown in figures 3 and 4). In addition to the configuration described and shown in figures 2-4, it is possible to have more than one expandable cannula 6 at the height of the centre of the metal part 3' or in other positions.

The operation of the saline infusion system of this second embodiment is as follows: first, the electrode is introduced in the tissue until reaching a desired position. In second place, the flexible internal probe 4' is pushed towards the outside a specific distance -between 1 and 5 mm- thus exceeding that same distance from the orifice 12 and therefore the external portion 6 having said length. In third place, before and during the operation of the cooled electrode, approximately 100 mL/hour of hypertonic saline solution -20% NaCl- is passed through the cannula 4' exiting through 6.

In other words, in both embodiments, the applicators are introduced in the centre of what one wants to be the thermal lesion (usually a tumour). The insertion of the applicator should be guided by imaging techniques (usually ultrasound or computed tomography).

In the first embodiment (fig. 1) the cooled electrode can be introduced in the tissue both before and simultaneously with the saline infusion system.

In the second embodiment (figs. 2-4) the infusion cannula is expanded within the tissue after having inserted the whole unit in the tissue. Likewise, said cannula should be retracted towards the interior of the electrode before withdrawing the unit from inside the tissue.

In both cases a temperature control system is added both inside and outside the radiofrequency generator and connected to a data acquisition system based, for example, on a computer.

The electrical variables (voltage, current and impedance) and thermal variables (temperature measured by thermocouple) are recorded by the radiofrequency generator or by another independent system.

The advantages of the applicator described in the present invention, as well as some of its specific characteristics can be found in the following experimental study on the optimal saline infusion distance during ablation with cooled electrodes.

Objective: Experimentally determining the optimal saline infusion distance in the tissue in terms of greater coagulation volume, greater coagulation diameters and improved energy deposition, all during the ablation using cooled electrodes.

Materials and methods of the experiments: The ablations were carried out at ambient temperature on beef livers obtained from a slaughter house. The electrodes were immersed to at least 1 cm below the surface of the tissue. The liver and the dispersive electrode (metal plate with surface area of 200 cm²) were immersed in a saline solution without direct contact between them.

All the experiments were carried out using a radiofrequency generator model CC-1 (Radionics, Burlington, Mass, USA) which is capable of delivering up to 2 A of 480 kHz sinusoidal current, which is equivalent to a power of 200 W on a charge of 50 ohms. Cooled electrodes model Cool-tip (Radionics, Valleylab, Boulder, CO, USA) were used with a 3 cm long metal point and a total thickness of 17G. The perfusion of saline to the tissue consisted of a 20% NaCl solution at ambient solution injected by an IPX1 pump (Alaris Medical Systems, Basingstoke, UK) with a 100 mL/h capacity. The infusion of this saline solution was carried out by a 14G infusion needle independent from the cooled electrode. This needle had an electrically insulating cover and was assembled in parallel to the electrode by a fixation system, i.e. the embodiment of the applicator device shown in figure 1 was used.

In all the experiments, the cooling of the Cool-Tip electrode and the infusion of saline towards the tissue started at least 60 seconds before ablation (and never more than 120 seconds). The ablation was carried out by the following power administration protocol: first minute at 50 W, second minute at 100 W, and maximum power after third minute. This protocol has been previously described, for example, in *"*Radiofrequency ablation of the porcine liver in vivo: increased coagulation with a cooled perfusion electrode" Lee JM, et al. Acad Radiol 2006; 13: 343-352. The radiofrequency generator continuously monitored the electrical impedance between the active electrode and the dispersive electrode. During the ablations, the administration of energy was interrupted during 1 minute (without perfusion of saline or cooling) in the case of a sudden increase of impedance above 200 ohms. Subsequently, the same power level was continued with.

A total of 35 ablations were performed, distributed in two experimental groups: the first consisted of 15 ablations of 10 minutes duration at three infusion distances 0, 2 and 4 mm. The second group consisted of 20 ablations of 20 minutes duration at three different infusion differences: 0, 2 and 4 mm.

In the statistical analysis of the results the continuous variables were compared by variance analysis and unpaired T test. Additionally, non-linear adjustments were carried out (i.e. regression models of superior order) and linear regression models with the object of finding an optimal infusion distance. The goodness of the adjustment was valued by the r² determination coefficient, which can be interpreted as the total observed variability which is explained by the model. Differences with a value of p<0.05 were considered statistically significant.

Results of the experiments: A macroscopic assay of the lesions obtained did not show the typical uniform ring of carbonized tissue observed in the lesions created with the cooled electrodes without perfusion of saline. This occurred despite the high quantity of power used. However, several degrees of carbonization in irregular areas (usually close to the trajectory of the electrode) were observed in 12 experiments. We should highlight that was carbonized tissue only observed in 2 experiments of the 2 mm infusion distance groups.

**Table 1. Effect of saline infusion distance in the tissue**

| Infusion distance | 0 | 2 | 4 | P* |
|---|---|---|---|---|
| Volume (cm³) Axial | 52.98 ± 15.04 | 91.75 ± 18.25 | 63.91 ± 11.75 | <0.05 |
| | | | | <0.05 |
| diameter (cm) | 6.25 ± 1.04 | 5.96 ± 0.23 | 5.25 ± 0.52 | <0.05 |
| Smaller transversal | 4.00 ± 0.40 | 5.28 ± 0.50 | 4.50 ± 0.83 | |
| diameter (cm) | 4.00 ± 0.37 | 5.52 ± 0.55 | 5.21 ± 0.51 | <0.05 |
| Larger transversal | | | | <0.01 |
| diameter (cm) | 166.34 ± 12.08 | 171.22 ± 9.80 | 151.27 ± 16.60 | |
| Power deposited (W) | | | | |

| | | | | |
|---|---|---|---|---|
| * Overall statistical significance | | | | |

The results (shown in Table 1) demonstrate that the saline infusion distance has a significant effect on both the lesion volume and the transversal lesion diameters (smaller and larger).

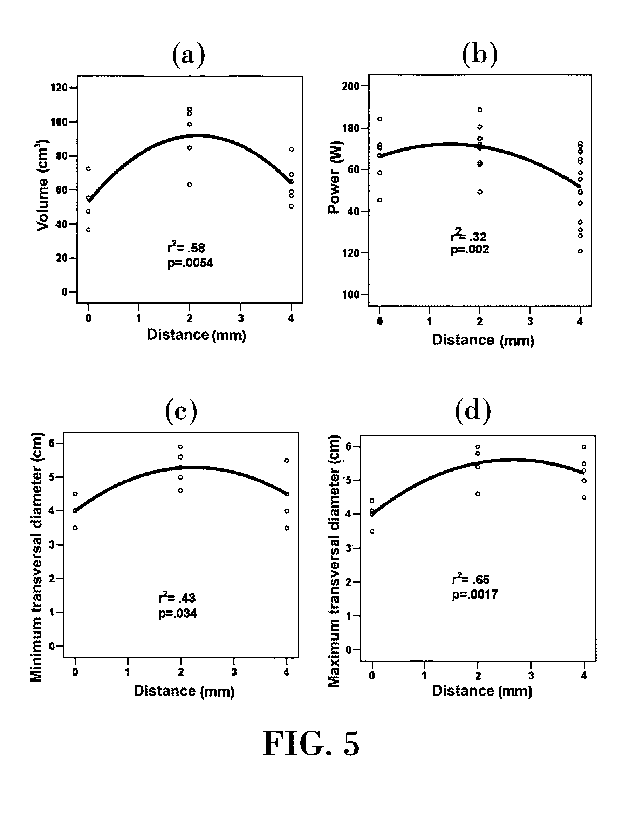

The best adjustments due to square minimums were always obtained using quadratic curves (shown in figure 5). In general, the best results were obtained for the infusion distance of 2 mm, permitting an increase in the lesion volume of at least 30% with respect to the 0 and 4 mm distance groups. Similarly, the 2 mm group showed an increase of at least 14% in the smaller transversal diameter with respect to the other two infusion distances.

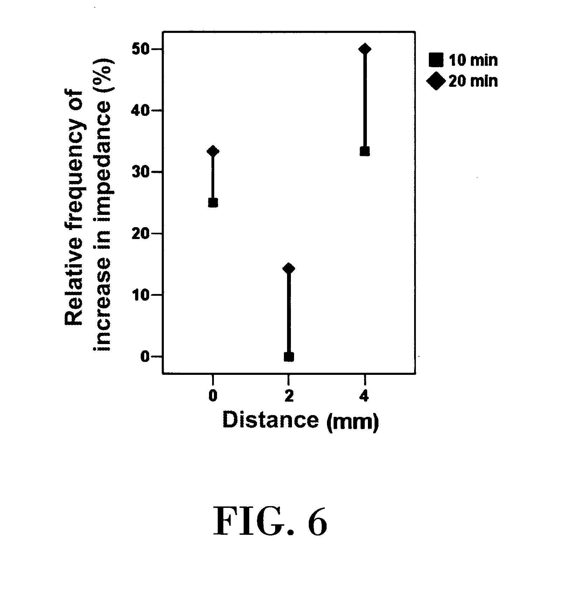

Figure 6 represents the relative frequency of increases in uncontrolled impedance for ablations of two durations (10 and 20 minutes) and for three infusion distances (0, 2 and 4 mm). Once more, the 2 mm distance showed a lower frequency.

Furthermore, the average deposition of energy throughout the ablation was also significantly greater in the case of 2 mm. Indeed, this result corresponds well with the relative high frequency of appearance of uncontrolled increases in impedance in the cases of 0 and 4 mm. Likewise, and as can be expected, the longer experiences (20 minutes) corresponded to a greater frequency of uncontrolled impedance increases.

Conclusions of the experiments: These results demonstrate that the optimum infusion distance of saline in the tissue during radiofrequency ablation with a cooled electrode would be around 2 mm, since it would permit obtaining lesions of greater volume and greater diameters. Therefore, the optimum distance is around 2 mm, although distances between 1 and 5 mm could be used.

The invention has also been described according to preferred embodiments thereof, but for persons skilled in the art it shall be evident that multiple variations can be introduced in said preferred embodiments without going outside the object of the invention claimed.

### APPLICATOR DEVICE FOR ABLATION BY RADIOFREQUENCY OF BIOLOGICAL TISSUES

### Description

### OBJECT OF THE INVENTION

The present invention relates in general to the therapeutic treatment of biological tissues, and to the use of thermal energy for the ablation of tumours.

### BACKGROUND OF THE INVENTION

The ablation of tumours by thermal damage to the unwanted cells by the application of radiofrequency currents is not new. When the frequency of the electrical current exceeds 10 kHz no pain or muscular contraction occurs, but instead ionic friction is generated which turns into heat. Radiofrequency ablation uses frequency currents between 100 kHz and 2 MHz. The increase in temperature in tissues above 50ºC leads to the rupture of proteins and membranes, which results in necrosis by coagulation and subsequent cell death. The use of radiofrequency ablation to destroy tumours is based on the doctor's skill in inserting one or several electrodes (also called applicators) in the tumour by guided computed tomography or ultrasounds. There has recently been great interest in radiofrequency ablation guided by image techniques as a new technique of minimally invasive therapy, especially for primary liver tumours or foci of metastasis, due to the high mortality and morbidity of standard surgical resection and the high number of patients who do not tolerate such an aggressive surgery.

In the most commonly used monopolar configuration, the current flows from an electrode with small dimensions called active electrode through the tissues towards a dispersive electrode (also called patch or plaque, of greater dimension, and located in the patient's back or thigh). During the conventional application of radiofrequency, the electrically power is fundamentally deposited in the tissue around the active electrode in a margin of a few millimetres, which produces heating exclusively localized in a narrow area around said electrode. This heat generated immediately adjacent to the electrode spreads through the rest of the tissue by thermal conduction. As a consequence, the application of radiofrequency involves a rapid increase in temperature (sometimes above 100ºC) in the tissue-active electrode interface, which causes the excessive desiccation of the tissue and formation of a necrosis by coagulation. The excessively desiccated cell tissue adheres to the electrode and forms an insulating cover which produces a fast and significant increase in electrical impedance. In these circumstances, the radiofrequency generator cannot distribute more power (this phenomenon is called "roll-off"). When this happens, it is not possible to deposit more energy and the total volume of the coagulated area is very limited, which may produce a lesion which does not include the entire tumour volume.

Different types of electrodes have been developed in order to avoid this problem. A strategy to minimize the problem of "roll-off' has been that adopted by cooled electrodes, such as that disclosed in the United States patent US-7077842-B1. This electrode is composed of two inner tubes connected in the distal part: one of them transports a cold saline fluid towards the distal point whilst the other returns that same fluid heated by the effect of heating of the tissue (which occurs in the same distal zone).

Also, for example, Spanish patent application ES-2229345 discloses a cooled electrode which includes a cavity in the main antenna or electrode which transports an infusion medium; it also has secondary antennas to house temperature sensors or administer radiofrequency energy.

There are other patents which disclose the use of conventional electrodes, cooled electrodes, and even groupings thereof, i.e. clusters such as those disclosed in United States patents US-6530922-B2 or US-6641580-B1. Patent US-6530922-B2 discloses a grouping or cluster of cooled electrodes; it also has a type of hook which protrudes laterally and whose function is to achieve a good anchoring with the tissue.

The use of this type of electrodes (*cooled electrodes*) achieves that the highly desiccated, even carbonized, area of tissue is not adjacent to the electrode, but instead moves to approximately 3 mm beyond the outer surface of the electrode, leaving a type of carbonized tissue of approximately 1 mm thickness. This localization of the carbonized tissue adapts well to the thermal profile of the tissue during the ablation with these electrodes, since the maximum temperature reached has been observed approximately 2.5 mm from the surface of the electrode. Beyond this point, the lesion is mainly created passively by heat conduction until equilibrium is reached. As a consequence, the cooled electrodes allow creating a greater coagulation volume with respect to conventional electrodes, but limited when it concerns ablating tumours of large dimensions.

Another strategy for increasing the coagulation volume is to infuse a saline solution within the tissue through the same active electrodes (they receive the name of wet electrodes). This strategy has been proposed in combination with monopolar or bipolar ablation electrodes, or in combination with expandable type electrodes (such as those disclosed in patent documents US-6660002-B1 and WO-2005/037118-A1). The result has been an increase in coagulation volume. Two mechanisms causing this event have been proposed: a) the high electrical conductivity of the saline solution -NaCl typically - produces an increase in the electrical conductivity of the infused tissue, which allows the deposition of a greater quantity of electrical power, and b) the infusion of fluid during the ablation improves heat conduction within the tissue, which permits the convection of the heat more effectively and rapidly on a large volume of tissue, thus avoiding the desiccation of tissue around the electrode. With these perfused electrodes, greater coagulation volumes have been achieved than with cooled electrodes. However, they still have some limitations, in particular, in a monopolar configuration:
a) The boiling of the saline solution in the active electrode-tissue interface still occurs frequently when the power is programmed above 50 watts. As a consequence of the vaporization of the saline around the electrode, cavitation may occur in the tissue adjacent to the electrode. This effect may bring three consequences. On the one hand, it may prevent the subsequent infusion of saline in the tissue, which makes the efficacy of the ablation decrease with this type of electrodes. On the other, it may cause the reflux of saline through the cavity created by the electrode in its entry trajectory in the tissue. This may also cause the leaking of the hot solution towards the peritoneal cavity. Finally, it may lead to the creation of an irregular coagulation volume which does not adapt to the form of the tumour.
b) Although boiling of the saline is avoided, it is difficult to ensure a controlled infusion thereof beyond 4 or 5 mm from the surface of the cooled electrode. This is especially true when the density of the tissue to be infused is high, and if the saline is infused through lateral orifices, and not through an orifice centrally located in the point of the electrode. As a consequence, the carbonization of the tissue may appear at 4-5 mm distance to the electrode, and therefore, interrupt the power deposition. Electrodes have also been proposed simultaneously combining cooling and infusion of saline such as those disclosed in the United States patent US-6514251-B1 and in the patent application US-2006/0122593A1. These electrodes are called cooled-wet. The most outstanding element of all of them is that the saline serum is infused through orifices located in the same surface of the cooled electrode, i.e. at zero distance from said electrode.

Another type of applicator has been disclosed in document WO-A-97/06739 (also published WO 97/06857). In addition to being cooled, this electrode permits the infusion of saline solution. This saline solution may run both through the cavity of the main electrode and through the cavity of the secondary probes or antennas. The secondary antennas do not infuse saline serum towards the tissue, instead the serum flows internally through two lumens. In this way, the secondary antennas may act as cooled electrodes (obviously distributing energy through them), in addition to being able to house temperature sensors.

Patent application EP-1656900-A2 discloses an applicator in the form of a loop designed to resect polyps which has a needle which infuses a serum at a distance (it can be used to dampen the polyp before resection). However, the loop electrode of this patient is a dry electrode, i.e. it is not a cooled electrode. Furthermore, this needle is not electrically insulated, instead it is metal which also allows it to act as active electrode. Finally, patent application US2006/178666 describes a device for ablating tissue in the living body which includes an elongate member defining a longitudinal passage having a distal opening and a proximal opening dimensioned to pass a guide element where the elongate member includes an electrode disposed at a distal portion of the elongate member and configured to be energized with high frequency energy to ablate tissue; nonetheless this document is silent about the configuration of the device just describing the parts of said device and the method of carrying out an ablation of biological tissue by means of said device, moreover the device is characterized by having an helical member extending from the distal end of an internal lumen.

As a consequence, it is desirable to have a device for the therapeutic treatment of biological tissue which exceeds the limitations of the aforementioned devices, thus achieving lesions with a high coagulation volume.

### DESCRIPTION OF THE INVENTION

The invention relates to an applicator device and to a method for radiofrequency ablation of biological tissues in accordance with claims 1 and 8, respectively. Preferred embodiments of the applicator device and of the method are defined in dependent claims.

The present invention provides an applicator device for radiofrequency ablation of biological tissues, particularly but not exclusively for hepatic tumours, which resolves the problems posed by the inventive subject included in the attached independent claims. The present invention achieves this objective as it combines the effect of the cooling in the electrode -by a closed circuit of cooling fluid-, with the infusion of a saline solution in the tissue at a critical point between 1 and 5 mm distance to the surface of the electrode through one or several infusing needles. In this way, the device in the present invention makes it possible to create lesions with greater coagulation volumes than those achieved by the use of conventional electrodes, cooled electrodes, and even groupings thereof. Although electrodes have also been proposed which combine cooling and saline infusion, they are electrodes which infuse the saline solution from the surface of the cooled electrode, and not at a critical distance between 1 and 5 mm, which does not allow improving the aforementioned limitations.

The applicator device object of the invention minimizes the boiling of the saline fluid and the carbonization of the adjacent tissue, improving the electrical conductivity in the critical area between 1 and 5 mm; in this way lesions are achieved with a high coagulation volume.

In accordance with a first aspect of the invention, it relates to an applicator device for radiofrequency ablation of biological tissues which comprises:
- an electrode whose outer surface is covered with an insulating cover in a proximal portion, and has a conductive distal portion, whose electrode includes cooling means, and
- an infusion system with a distal end for infusing a conductive fluid (such as, for example, a saline solution) in said tissue, characterized in that said distal end of the infusion system has an electrically insulating outer cover and is located, in the infusion position of said fluid, at a distance between 1 and 5 mm from said outer surface of the conductive distal portion of the cooled electrode.

In accordance with a preferred embodiment of the applicator device of the invention, the infusion system comprises at least one needle -with electrically insulating cover- which, in the use position, is located fixed to the device, and a distal end of said needle is located at said distance between 1 and 5 mm from the outer surface of the distal portion of the electrode. In this embodiment the axial axis of said needle and that of the electrode are preferably parallel.

In accordance with another possible embodiment of the applicator device of the invention, the infusion system comprises at least one expandable tube with a distal end -whose outer surface has an electrically insulating cover-, and in that said distal end in the infusion position of the conductive fluid is at a distance between 1 and 5 mm from the outer surface of the distal portion of the electrode. In this case, said expandable tube preferably is located in a channel in the interior of the electrode.

Preferably, the distal end of the infusion system is located, in the infusion position of the conductive fluid, at a height corresponding to half of said conductive distal portion of the cooled electrode.

In accordance with a second aspect of the invention, it relates to a method of radiofrequency ablation of biological tissues which comprises:
- inserting in said biological tissue a conductive distal portion of an electrode,
- supplying high frequency electrical energy in said distal portion of the electrode,
- cooling said electrode continuously during said supply of energy and also optionally before said supply, and
- injecting in said biological tissue a conductive fluid at a distance between 1 and 5 mm from an outer surface of a conductive distal portion of said cooled electrode.

Preferably said fluid is inserted in the biological tissue by an infusion system, which is also inserted in the biological tissue and is externally covered by an electrically insulating cover, and simultaneously or previously the cooled electrode is inserted.

A single applicator can also be inserted in the biological tissue which comprises the electrode and an infusion system of said conductive fluid. In said case, said infusion system comprises at least one expandable tube - whose outer surface has an electrically insulating cover- and in that injecting the conductive fluid in the biological tissue comprises expanding said tube until a distal end of said tube is at a distance between 1 and 5 mm from the outer surface of the electrode.

In the infusion position of the conductive fluid the distal end of the infusion system is located preferably at a height corresponding to half of said conductive distal portion of the cooled electrode.

### BRIEF DESCRIPTION OF THE DRAWINGS

To complement the description being made, and in order to aid towards a better understanding of the characteristics of the invention, in accordance with preferred examples of practical embodiment thereof, a set of drawings is attached as an integral part of said description wherein the following has been represented with illustrative and non-limitative character:
Figure 1 shows a partial sectional view of an applicator device for ablation of biological tissues in accordance with a first possible embodiment thereof.
Figure 2 shows a partial sectional view of a second possible embodiment of the applicator device of the invention.
Figures 3 and 4 are two perspective views of the device of the invention according to the preferred embodiment shown in figure 2.
Figure 5 shows the effect of the saline infusion distance on the lesion volume (a), average distributed power (b), smaller or minimum diameters (c) and larger or maximum diameters (d).
Figure 6 represents the relative frequency of uncontrolled increases in impedance according to the ablation time and the infusion distance.

### DESCRIPTION OF A PREFERRED EMBODIMENT OF THE INVENTION

Figure 1 shows a cooled electrode with a saline infusion system assembled in parallel by a fixating system.

This applicator 1 contains a cooled electrode and a saline infusion system assembled in parallel by means of a fixation system 10 and at a distance between them which can vary between 1 and 5 mm.

The electrode's structure is as follows: it has an external diameter of approximately 1.8 mm. It is made of stainless steel and has a part covered with electrical insulator 2, and has a sharp point at its end 5. It can also have one or more thermocouples 8 located in the central part of the metal zone 3 and adhered to the interior of the electrode. The length of the metal zone should adapt to the length of the tissue area one wants to coagulate. The cooling system is based on a cannula 7 which is approximately 0.4 mm in diameter. In figure 1 the solid arrows indicate the direction of the cooled fluid which circulates internally through the cooled electrode. The broken arrow indicates the direction of the infused fluid directly in the tissue at a critical point between 1 and 5 mm from the cover of the cooled electrode.

Once a dispersive electrode (not represented) is located on the back or thigh of the patient and connected to a radiofrequency generator by a monopolar configuration, the operation is as follows: first, the cooled electrode is introduced in the desired position of the tissue. In second place, water or pre-cooled saline solution is infused (at a temperature, for example, of 0ºC) through the cannula 7 and at a flow of approximately 30 mL/minute. This is done, for example, by a peristaltic pump. The temperature of the metal zone 3 of the electrode is continuously monitored by the thermocouple 8 and displayed by the system of the radiofrequency generator (such as, for example, that marketed by Radionics Inc.) or by any independent system built for said purpose. When the temperature recorded by the thermocouple 8 reaches a value under 20ºC the distribution of radiofrequency energy starts through the metal part 3 following a specific energy deposition algorithm. During said energy distribution, the internal infusion of saline or pre-cooled water does not cease. The energy distribution algorithm may be controlled automatically or manually. There are different algorithm alternatives, but they are typically based on an energy distribution at maximum power (usually 200 watts) and an interruption -lasting between 10 and 60 seconds- in the case that the electrical impedance (measured between the metal part 3 and the dispersive electrode) exceeds a level -between 10 and 30 ohms- the initial impedance. In this configuration said interruption may also take place in the event that the temperature recorded in the thermocouple 8 should exceed 30ºC. Throughout the method, the cold liquid that flows through the cannula 7 is returned, now heated, towards a reservoir (not shown) for said purpose.

The objectives of the cooled electrode are:
- distributing the energy through its metal part directly towards the biological tissue;
- minimizing the carbonization of the tissue adjacent to the electrode; and,
- minimizing the boiling of the saline fluid diffused in the tissue adjacent to the electrode.

The saline infusion system is based on a cannula 4 of approximately 1 mm of diameter with insulating cover and assembled in parallel to the cooled electrode. The distance from the exit point of the saline from the cannula's 4 to the metal part 3 of the electrode may vary between 1 and 5 mm. The infusion system may be introduced in the tissue at the same time as the electrode or *a posteriori.* One minute before the power is distributed through the metal part 3 of the electrode, and also during said distribution, an infusion of isotonic (0.9%) or hypertonic (typically 20% NaCl) saline solution is performed through the cannula 4 at a flow of approximately 100 mL/hour.

The objectives of the saline infusion system are:
- improving the electrical conductivity of the tissue by its high NaCl concentration (it is a saline with a typical concentration between 0.9 and 40% NaCl); and,
- cooling the tissue located at a critical distance between 1 and 5 mm from the metal part 3 of the electrode in order to avoid the carbonization of said tissue.

For its part, the fixation system 10 maintains the cannula 4 of the infusion system at a specific distance -between 2 and 5 mm- from the outer part of the electrode. Different configurations are possible, the simplest would be based on a resin block that solidly joins the electrode and the cannula (4).

Figures 2-4 show a second embodiment of the invention, with a cooled electrode with expandable tubes of saline solution all forming a single applicator 1 '.

In this embodiment, a cooled electrode and infusion system are incorporated in the same element which serves as a handle 9.

The objectives in this case are the same as those stated for the embodiment shown in figure 1.

In this case, the maximum outer diameter (it could be less) is approximately 2.2 mm. The electrode is made from stainless steel and has a part with insulating cover 2' and a metal part 3' finished in a point 5'. The length of the metal zone 3' should adapt to the length of the tissue area one wants to coagulate. It has an internal infusion T cannula 7'-of approximately 0.4 mm in diameter. There is also a thermocouple 8' in the inner wall of the electrode at the average distance of the metal part 3'.

The operation in this case is the same as has been described for the device 1 shown in figure 1.

In this case, the saline infusion system comprises one or more expandable tubes of saline infusion 6. The objectives of this saline infusion system are the same as that described for the embodiment of figure 1, and also the following:
- avoiding a double puncture in the tissue, as required if the first embodiment is used; and,
- infusing the saline (isotonic or hypertonic) at a specific distance with greater accuracy.

The system comprises a saline infusion cannula 4' which is incorporated within a rigid channel 11 located inside the electrode. It has an approximate diameter of 1 mm. This cannula 4' ends up exiting the electrode through a lateral orifice 12 located in the centre of the metal part 3'. Through this orifice the cannula exits the electrode forming an external cannula 6 that can adopt different forms such as straight (shown in figure 2), or J-shaped (shown in figures 3 and 4). In addition to the configuration described and shown in figures 2-4, it is possible to have more than one expandable cannula 6 at the height of the centre of the metal part 3' or in other positions.

The operation of the saline infusion system of this second embodiment is as follows: first, the electrode is introduced in the tissue until reaching a desired position. In second place, the flexible internal probe 4' is pushed towards the outside a specific distance -between 1 and 5 mm- thus exceeding that same distance from the orifice 12 and therefore the external portion 6 having said length. In third place, before and during the operation of the cooled electrode, approximately 100 mL/hour of hypertonic saline solution -20% NaCl- is passed through the cannula 4' exiting through 6.

In other words, in both embodiments, the applicators are introduced in the centre of what one wants to be the thermal lesion (usually a tumour). The insertion of the applicator should be guided by imaging techniques (usually ultrasound or computed tomography).

In the first embodiment (fig. 1) the cooled electrode can be introduced in the tissue both before and simultaneously with the saline infusion system.

In the second embodiment (figs. 2-4) the infusion cannula is expanded within the tissue after having inserted the whole unit in the tissue. Likewise, said cannula should be retracted towards the interior of the electrode before withdrawing the unit from inside the tissue.

In both cases a temperature control system is added both inside and outside the radiofrequency generator and connected to a data acquisition system based, for example, on a computer.

The electrical variables (voltage, current and impedance) and thermal variables (temperature measured by thermocouple) are recorded by the radiofrequency generator or by another independent system.

The advantages of the applicator described in the present invention, as well as some of its specific characteristics can be found in the following experimental study on the optimal saline infusion distance during ablation with cooled electrodes.

Objective: Experimentally determining the optimal saline infusion distance in the tissue in terms of greater coagulation volume, greater coagulation diameters and improved energy deposition, all during the ablation using cooled electrodes.

Materials and methods of the experiments: The ablations were carried out at ambient temperature on beef livers obtained from a slaughter house. The electrodes were immersed to at least 1 cm below the surface of the tissue. The liver and the dispersive electrode (metal plate with surface area of 200 cm²) were immersed in a saline solution without direct contact between them.

All the experiments were carried out using a radiofrequency generator model CC-1 (Radionics, Burlington, Mass, USA) which is capable of delivering up to 2 A of 480 kHz sinusoidal current, which is equivalent to a power of 200 W on a charge of 50 ohms. Cooled electrodes model Cool-tip (Radionics, Valleylab, Boulder, CO, USA) were used with a 3 cm long metal point and a total thickness of 17G. The perfusion of saline to the tissue consisted of a 20% NaCl solution at ambient solution injected by an IPX1 pump (Alaris Medical Systems, Basingstoke, UK) with a 100 mL/h capacity. The infusion of this saline solution was carried out by a 14G infusion needle independent from the cooled electrode. This needle had an electrically insulating cover and was assembled in parallel to the electrode by a fixation system, i.e. the embodiment of the applicator device shown in figure 1 was used.

In all the experiments, the cooling of the Cool-Tip electrode and the infusion of saline towards the tissue started at least 60 seconds before ablation (and never more than 120 seconds). The ablation was carried out by the following power administration protocol: first minute at 50 W, second minute at 100 W, and maximum power after third minute. This protocol has been previously described, for example, in *"*Radiofrequency ablation of the porcine liver in vivo: increased coagulation with a cooled perfusion electrode" Lee JM, et al. Acad Radiol 2006; 13: 343-352. The radiofrequency generator continuously monitored the electrical impedance between the active electrode and the dispersive electrode. During the ablations, the administration of energy was interrupted during 1 minute (without perfusion of saline or cooling) in the case of a sudden increase of impedance above 200 ohms. Subsequently, the same power level was continued with.

A total of 35 ablations were performed, distributed in two experimental groups: the first consisted of 15 ablations of 10 minutes duration at three infusion distances 0, 2 and 4 mm. The second group consisted of 20 ablations of 20 minutes duration at three different infusion differences: 0, 2 and 4 mm.

In the statistical analysis of the results the continuous variables were compared by variance analysis and unpaired T test. Additionally, non-linear adjustments were carried out (i.e. regression models of superior order) and linear regression models with the object of finding an optimal infusion distance. The goodness of the adjustment was valued by the r² determination coefficient, which can be interpreted as the total observed variability which is explained by the model. Differences with a value of p<0.05 were considered statistically significant.

Results of the experiments: A macroscopic assay of the lesions obtained did not show the typical uniform ring of carbonized tissue observed in the lesions created with the cooled electrodes without perfusion of saline. This occurred despite the high quantity of power used. However, several degrees of carbonization in irregular areas (usually close to the trajectory of the electrode) were observed in 12 experiments. We should highlight that was carbonized tissue only observed in 2 experiments of the 2 mm infusion distance groups.

**Table 1. Effect of saline infusion distance in the tissue**

| Infusion distance | 0 | 2 | 4 | P* |
|---|---|---|---|---|
| Volume (cm³) Axial | 52.98 ± 15.04 | 91.75 ± 18.25 | 63.91 ± 11.75 | <0.05 |
| | | | | <0.05 |
| diameter (cm) | 6.25 ± 1.04 | 5.96 ± 0.23 | 5.25 ± 0.52 | <0.05 |
| Smaller transversal | 4.00 ± 0.40 | 5.28 ± 0.50 | 4.50 ± 0.83 | |
| diameter (cm) | 4.00 ± 0.37 | 5.52 ± 0.55 | 5.21 ± 0.51 | <0.05 |
| Larger transversal | | | | <0.01 |
| diameter (cm) | 166.34 ± 12.08 | 171.22 ± 9.80 | 151.27 ± 16.60 | |
| Power deposited (W) | | | | |

| | | | | |
|---|---|---|---|---|
| * Overall statistical significance | | | | |

The results (shown in Table 1) demonstrate that the saline infusion distance has a significant effect on both the lesion volume and the transversal lesion diameters (smaller and larger).

The best adjustments due to square minimums were always obtained using quadratic curves (shown in figure 5). In general, the best results were obtained for the infusion distance of 2 mm, permitting an increase in the lesion volume of at least 30% with respect to the 0 and 4 mm distance groups. Similarly, the 2 mm group showed an increase of at least 14% in the smaller transversal diameter with respect to the other two infusion distances.

Figure 6 represents the relative frequency of increases in uncontrolled impedance for ablations of two durations (10 and 20 minutes) and for three infusion distances (0, 2 and 4 mm). Once more, the 2 mm distance showed a lower frequency.

Furthermore, the average deposition of energy throughout the ablation was also significantly greater in the case of 2 mm. Indeed, this result corresponds well with the relative high frequency of appearance of uncontrolled increases in impedance in the cases of 0 and 4 mm. Likewise, and as can be expected, the longer experiences (20 minutes) corresponded to a greater frequency of uncontrolled impedance increases.

Conclusions of the experiments: These results demonstrate that the optimum infusion distance of saline in the tissue during radiofrequency ablation with a cooled electrode would be around 2 mm, since it would permit obtaining lesions of greater volume and greater diameters. Therefore, the optimum distance is around 2 mm, although distances between 1 and 5 mm could be used.

The invention has also been described according to preferred embodiments thereof, but for persons skilled in the art it shall be evident that multiple variations can be introduced in said preferred embodiments without going outside the object of the invention claimed.

## Claims

1. An applicator device (1, 1') for radiofrequency ablation of biological tissues, which comprises:
- an electrode whose outer surface is covered with an insulating cover (2, 2') in a proximal portion, and has a conductive distal portion (3, 3'), whose electrode includes cooling means (7, 7'), and
- an infusion system with a distal end (13, 6) for infusing a conductive fluid in said tissue,
**characterized in that**
said distal end (13, 6) of the infusion system has an electrically insulating outer cover and is located, in an infusion position for said conductive fluid, at a distance between 2 and 5 mm from the outer surface of the conductive distal portion of the cooled electrode and at a longitudinal half of said conductive distal portion (3, 3') of the electrode.

2. Applicator device according to claim 1, **characterized in that** said infusion system comprises at least one needle (4) with electrically insulating cover which, in the use position, is located fixed to the device, and a distal end (13) of said needle being located at said distance between 2 and 5 mm from the outer surface of the distal portion of the electrode.

3. Applicator device according to claim 2, **characterized in that** the axial axis of said needle is parallel to the axial axis of the electrode.

4. Applicator device according to claim 1, **characterized in that** said infusion system comprises at least one expandable tube (4') with a distal end (6) whose outer surface has an electrically insulating cover, and **in that** said distal end (6) in the infusion position for the conductive fluid is at said distance between 2 and 5 mm from the outer surface of the distal portion of the electrode.

5. Applicator device according to claim 4, **characterized in that** said expandable tube (4') is located in a channel (11) in the interior of the electrode.

6. Applicator device according to any of the preceding claims, **characterized in that** said conductive fluid is a saline solution.

## Patentansprüche

1. Eine Applikatorvorrichtung (1, 1') zur Radiofrequenzablation biologischer Gewebe, die Folgendes umfasst:
- eine Elektrode, deren äußere Oberfläche mit einer isolierenden Abdeckung (2, 2') in einem proximalen Teil bedeckt ist und die einen leitfähigen distalen Teil (3, 3') besitzt, wobei die Elektrode Kühlmittel (7, 7') einschließt, und
- ein Infusionssystem mit einem distalen Ende (13, 6) zur Einflößung eines leitfähigen Fluidums in dieses Gewebe,
**dadurch gekennzeichnet, dass** dieses distale Ende (13, 6) des Infusionssystems eine elektrisch isolierende äußere Abdeckung besitzt und sich in einer Infusionsposition dieses leitfähigen Fluidums in einem Abstand zwischen 2 und 5 mm von der äußeren Oberfläche des leitfähigen distalen Teils der gekühlten Elektrode und an einer Längshälfte des leitfähigen distalen Teils (3, 3') der Elektrode befindet.

2. Applikatorvorrichtung nach Anspruch 1, **dadurch gekennzeichnet, dass** dieses Infusionssystem mindestens eine Nadel (4) mit einer elektrisch isolierenden Abdeckung besitzt, die sich in der Einsatzposition an der Vorrichtung fixiert befindet, und wobei ein distales Ende (13) dieser Nadel sich in diesem Abstand von 2 bis 5 mm von der äußeren Oberfläche des distalen Teils der Elektrode entfernt befindet.

3. Applikatorvorrichtung nach Anspruch 2, **dadurch gekennzeichnet, dass** die axiale Achse dieser Nadel parallel zur axialen Achse der Elektrode verläuft.

4. Applikatorvorrichtung nach Anspruch 1, **dadurch gekennzeichnet, dass** die besagte Infusionsvorrichtung mindestens ein expandierbares Rohr (4') mit einem distalen Ende (6) umfasst, dessen äußere Oberfläche eine elektrisch isolierende Abdeckung besitzt, und dadurch, dass dieses distale Ende (6) sich in der Infusionsposition des leitfähigen Fluidums in diesem Abstand zwischen 2 und 5 mm von der äußeren Oberfläche des distalen Teils der Elektrode entfernt befindet.

5. Applikatorvorrichtung nach Anspruch 4, **dadurch gekennzeichnet, dass** sich das expandierbare Rohr (4') in einem Kanal (11) im Inneren der Elektrode befindet.

6. Applikatorvorrichtung nach jedem der vorherigen Ansprüche, **dadurch gekennzeichnet, dass** es sich bei diesem leitfähigen Fluidum um eine Salzlösung handelt.

## Revendications

1. Un dispositif applicateur (1, 1') pour l'ablation par radiofréquence de tissus biologiques, qui comprend :
- une électrode dont la surface externe est recouverte d'un revêtement isolant (2, 2') sur une portion proximale, et possède une portion distale conductrice (3, 3'), dont l'électrode comprend des moyens de refroidissement (7, 7'), et
- un système de perfusion avec une extrémité distale (13, 6) pour perfuser un fluide conducteur dans ledit tissu,
**caractérisé en ce que** ladite extrémité distale (13, 6) du système de perfusion possède un revêtement externe d'isolation électrique et se situe, dans une position de perfusion pour ledit fluide conducteur, à une distance comprise entre 2 et 5 mm de la surface externe de la portion distale conductrice de l'électrode refroidie et à la moitié dans le sens longitudinal de ladite portion distale conductrice (3, 3') de l'électrode.

2. Dispositif applicateur selon la revendication 1, **caractérisé en ce que** ledit système de perfusion comprend au moins une aiguille (4) avec revêtement d'isolation électrique qui, dans la position d'utilisation, se trouve fixée au dispositif, et une extrémité distale (13) de ladite aiguille se situant à ladite distance comprise entre 2 et 5 mm de la surface externe de la portion distale de l'électrode.

3. Dispositif applicateur selon la revendication 2, **caractérisé en ce que** l'axe axial de ladite aiguille est parallèle à l'axe axial de l'électrode.

4. Dispositif applicateur selon la revendication 1, **caractérisé en ce que** ledit système de perfusion comprend au moins un tube extensible (4') avec une extrémité distale (6) dont la surface externe possède un revêtement d'isolation électrique, et **en ce que** ladite extrémité distale (6) dans la position de perfusion pour le fluide conducteur est à ladite distance comprise entre 2 et 5 mm de la surface externe de la portion distale de l'électrode.

5. Dispositif applicateur selon la revendication 4, **caractérisé en ce que** ledit tube extensible (4') est situé dans un canal (11) à l'intérieur de l'électrode.

6. Dispositif applicateur selon n'importe laquelle des revendications précédentes, **caractérisé en ce que** ledit fluide conducteur est une solution saline.
